# FASCICULE DE BREVET EUROPEEN

(11) **EP 2 978 815 B1**
(45) Date de publication et mention de la délivrance du brevet: **05.12.2018**
(21) Numéro de dépôt: 14720179.2
(22) Date de dépôt: 28.03.2014
(51) Int. Cl.: C09D 103/12, C08L 3/12

(54) **COMPOSITIONS FILMOGÈNES POUR LE PELLICULAGE DE FORMES SOLIDES**
FILMBILDENDE ZUSAMMENSETZUNGEN ZUR FILMLACKIERUNG FESTER FORMEN
FILM-FORMING COMPOSTIONS FOR THE FILM-COATING OF SOLID FORMS

(30) Priorité: 29.03.2013 FR 1352880
(43) Date de publication de la demande: 03.02.2016
(73) Titulaire: Roquette Frères, 62136 Lestrem (FR)
(72) Inventeur: PARISSAUX, Xavier, F-62120 Campagne Les Wardrecques (FR); LE BIHAN, Grégory, F-62232 Annezin (FR); CROQUET, Sébastien, F-59660 Merville (FR)
(74) Mandataire: Cabinet Plasseraud
(86) Numéro de dépôt international: PCT/FR2014/050737
(87) Numéro de publication internationale: WO 2014/155015

(56) Documents cités:
- WO-A1-02/00205
- WO-A1-02/092708
- WO-A2-01/91721
- WO-A2-01/92400
- WO-A2-01/92401

## Description

La présente invention a pour objet une composition filmogène comprenant un amidon hydroxypropylé fluidifié et du sorbitol, et vise également un procédé de pelliculage utilisant une telle composition.

La présente invention a également pour objet des formes solides pelliculées comprenant un amidon hydroxypropylé fluidifié et du sorbitol dans leur pelliculage.

### ETAT DE L'ART

Les compositions filmogènes sont très utilisées dans l'industrie pharmaceutique, cosmétique et agroalimentaire, notamment pour le pelliculage de formes solides.

Le pelliculage de formes solides, en particulier de comprimés, est une opération qui vise à obtenir une protection physique et chimique des ingrédients actifs. Le médicament est ainsi protégé de son environnement (humidité, oxygène, lumière). Le pelliculage permet également de masquer le goût, l'odeur ou la couleur des ingrédients actifs, et permet aussi de modifier leur vitesse de libération dans l'organisme en augmentant la résistance du comprimé aux sucs gastriques. Le pelliculage facilite l'ingestion des comprimés, améliore leur apparence et leur intégrité mécanique. La plupart des formes solides peuvent être pelliculées : comprimés, granulés, gélules, semences, confiseries.

Le pelliculage consiste en l'application d'une composition liquide filmogène sur une forme solide, cette composition devenant après séchage un film protecteur.

L'agent filmogène idéal doit permettre l'obtention d'un film élastique et cohésif. Il est de préférence soluble dans l'eau, l'eau étant le solvant préféré par opposition aux solvants organiques en raison de sa facilité d'utilisation et de son innocuité.

Afin de rendre les films moins cassants, on intègre parfois un plastifiant à ces compositions filmogènes. Le plastifiant abaisse la température de transition vitreuse (Tg) du film, il augmente sa flexibilité, sa résistance et sa résilience, et facilite la manipulation de la composition filmogène.

L'amidon est très utilisé dans l'industrie textile et papetière comme agent filmogène. Il l'est en revanche beaucoup moins pour des applications dans le domaine du pelliculage.

Malgré des contraintes à la rupture comparables à celles de certains polymères synthétiques, les matériaux à base d'amidon restent en effet très fragiles. Ils sont en plus très sensibles à l'eau : la quantité d'eau contenue dans les films modifie de manière très importante la Tg et donc les propriétés mécaniques des matériaux obtenus. D'autres facteurs tels que la masse molaire et la cristallinité peuvent jouer un rôle sur les propriétés des films à base d'amidon.

Une autre difficulté est que dans le domaine particulier du pelliculage, les films sont très fins, de l'ordre de quelques dizaines de micromètres, et sont soumis à des contraintes particulièrement importantes, qui vont favoriser leur fissuration.

Une fissuration du film se produit en particulier lorsque celui-ci est trop déshydraté, par exemple lors du séchage rapide des comprimés au moment du pelliculage, ou postérieurement, dans le cas de comprimés qui accaparent l'eau contenue dans le film.

Le film peut également casser au cours du stockage, lorsque les conditions ambiantes favorisent le gonflement des comprimés, par exemple lorsqu'ils sont placés en atmosphère humide, à température élevée, et en particulier lorsqu'ils ne possèdent pas d'emballage. Ceci est particulièrement vrai pour les comprimés hygroscopiques, dont le volume augmente de manière significative par absorption de l'humidité de l'air.

Il faut par ailleurs que la composition filmogène puisse répondre aux autres contraintes liées à la problématique du pelliculage, notamment qu'elle puisse adhérer aux formes solides à pelliculer, qu'elle soit non réactive vis-à-vis des ingrédients qui les composent, et qu'elle présente un comportement adapté pour une conduite machine (par exemple en terme de viscosité ou de Tg).

Une autre difficulté est que les formes solides à pelliculer diffèrent en composition et en forme et peuvent donc présenter des caractéristiques physico-chimiques extrêmement variées par exemple pour ce qui est du caractère hygroscopique, de la dureté, de la porosité, de l'énergie de surface, ou encore de la solubilité. Il est donc particulièrement difficile de trouver des compositions filmogènes adaptées au pelliculage de toutes ces formes solides.

Il n'existe pas à l'heure actuelle de composition filmogène amylacée qui réponde de manière efficace à tous ces problèmes. Il n'existe pas de composition filmogène « universelle » pour une application dans le pelliculage.

Or, il est du mérite de la Demanderesse d'avoir réussi à développer des compositions filmogènes particulièrement bien adaptées pour le pelliculage de toutes sortes de formes solides. Les phénomènes de fissuration des films sont largement diminués, voire supprimés, et ce même pour des formes solides dont le volume varie fortement du fait de la reprise en eau ou d'une expansion thermique.

La solution repose sur l'identification judicieuse de couples particuliers amidon / plastifiant qui permettent d'obtenir des résultats exceptionnels en termes de plastification. L'amidon sélectionné par la Demanderesse est en particulier un amidon hydroxypropylé fluidifié, et le plastifiant utilisé est le sorbitol.

Les résultats obtenus par la Demanderesse en choisissant le sorbitol comme plastifiant et un amidon hydroxypropylé fluidifié comme agent filmogène sont exceptionnels, et s'opposent à ce que la littérature enseigne.

Premièrement, les amidons fluidifiés sont connus pour leur tendance à former des films plus cassants que les amidons natifs, du fait de leur poids moléculaire plus faible.

D'un autre côté, le sorbitol est considéré depuis plus de dix ans comme un mauvais plastifiant de l'amidon. Comparé au glycérol, classiquement employé, la littérature enseigne que le sorbitol aboutit à la formation de films plus rigides et plus friables, ce qui a conforté jusqu'à présent l'homme du métier dans le choix du glycérol comme plastifiant pour les matières amylacées.

On peut par exemple citer une étude de Yachuan Zhang et al. (Plasticization of Pea Starch Films with Monosaccharides and Polyols. Journal of food science, Vol. 71, Nr. 6 ; 253-261 (2006)) sur la plastification d'un amidon de pois, et une étude de Aji P. Mathew et al (Plasticized waxy maize starch : effect of polyols and relative humidity on material properties. Biomacromolecules, 3.1101-1108 (2002)), sur la plastification d'un amidon de maïs cireux. Ces études confirment la supériorité du glycérol dans la plastification de matières amylacées.

D'autres études ont même montré que lorsque le sorbitol est utilisé en dessous de certaines teneurs, il exerce un effet anti-plastifiant.

Par exemple, une étude de S. Gaudin et al. (Plasticisation and mobility in starch-sorbitol films. Journal of Cereal Science 29. 173-284 (1999)) décrit l'effet anti-plastifiant du sorbitol sur l'amidon de blé lorsqu'il est utilisé à une teneur inférieure à 27% en poids par rapport au poids d'amidon.

Une étude de Suzana Mali et al (Antiplasticizing effect of glycerol and sorbitol on the properties of cassava starch films. Braz. J. Food Technol., Vol. 11, n°3, 194-200 (2008)), décrit l'effet anti-plastifiant du sorbitol sur l'amidon de cassava à une teneur inférieure à 15% en poids, par rapport au poids d'amidon.

Or la Demanderesse a montré qu'en choisissant un amidon hydroxypropylé fluidifié comme agent filmogène, le sorbitol exerçait son effet plastifiant même en dessous de ces teneurs, démontrant ainsi la particularité du couple amidon / plastifiant identifié par la Demanderesse.

Il existe par ailleurs quelques documents qui relatent l'utilisation de matières amylacées pour des applications dans le pelliculage.

Dans son brevet français FR 2 862 654 B1 par exemple, la Demanderesse décrit une composition de pelliculage utilisant un amidon de légumineuse stabilisé plastifié avec du glycérol. Ces compositions possédaient déjà de bonnes propriétés comparativement à celles de l'art antérieur. Cependant, la Demanderesse a mis en évidence des problèmes de fissuration des films qui apparaissaient dans certains cas, notamment sur des comprimés très hygroscopiques.

Le sorbitol d'autre part a été brièvement décrit pour son utilisation dans des compositions de pelliculage.

On peut par exemple citer la demande WO02/00205 sur des compositions utilisant un amidon de maïs cireux acétylé. Il ressort de cette demande qu'aucun test de pelliculage de formes solides n'est donné pour ces compositions, car le plastifiant préférentiel était encore une fois le glycérol.

La demande de brevet WO 02/092708 décrit également des compositions filmogènes amylacées comprenant du sorbitol, utilisant un amidon de maïs à haute teneur en amylose comme agent filmogène. Cependant, le sorbitol était toujours utilisé en combinaison avec le glycérol, et à des teneurs extrêmement élevées. Le sorbitol et le glycérol représentaient ainsi chacun 50% du poids d'amidon, c'est-à-dire que ces compositions comprenaient autant de plastifiant que d'amidon.

Ceci démontre encore la particularité de l'association amidon / plastifiant identifiée par la Demanderesse, puisque de très bons résultats ont été obtenus, même en utilisant des teneurs en sorbitol largement inférieures.

### BUTS DE L'INVENTION

Ainsi la présente invention a pour object de fournir de nouvelles compositions amylacées filmogènes, notamment pour le pelliculage de formes solides, qui permettent de diminuer, voire de supprimer le problème de fissuration des films.

L'invention permet de répondre aux problèmes susmentionnés en fournissant des compositions filmogènes faciles à préparer et à mettre en oeuvre, d'un coût raisonnable, faisant appel à des matériaux bio-sourcés, sûrs du point de vue de la santé des manipulateurs et des consommateurs.

### DESCRIPTION

Comme montré dans l'Exemple 1, et contrairement à ce qu'enseigne l'art antérieur, le sorbitol permet d'abaisser la Tg du film amylacé à des teneurs de l'ordre de 5% en poids par rapport au poids d'amidon. Contrairement à ce qui avait donc été constaté auparavant, dans le cas particulier de l'amidon choisi selon la présente invention, non seulement le sorbitol exerce un effet plastifiant à de faibles teneurs, mais il est en plus un meilleur plastifiant que le glycérol. En effet de telles performances ne sont pas constatées dès lors que l'on modifie l'amidon ou le plastifiant utilisé, ce qui démontre l'importance du choix judicieux du couple amidon / plastifiant identifié par la Demanderesse.

La pertinence de ces faibles teneurs a été confirmée pour des applications dans le pelliculage. Pour des teneurs en plastifiant de l'ordre de 8,6% en poids par rapport au poids d'amidon, les compositions filmogènes selon l'invention ont conduit à la formation d'une couche de pelliculage uniforme et adhérente lorsque le sorbitol était utilisé, alors que des fissurations des films de pelliculage se sont produites avec le glycérol (Exemple 2.a)).

Dans les compositions filmogènes selon l'invention, le sorbitol peut ainsi être introduit dans des teneurs comprises entre 5 et 80% en poids par rapport au poids d'amidon hydroxypropylé fluidifié, en particulier entre 5 et 40% pour des applications dans le pelliculage.

Le sorbitol et l'amidon hydroxypropylé fluidifié selon l'invention présentent par ailleurs l'avantage de pouvoir être mélangés sous forme pulvérulente. En effet, les plastifiants auparavant utilisés dans les compositions filmogènes, notamment le glycérol, se présentaient toujours sous forme liquide. Or l'amidon ne peut être mélangé à l'eau qu'au moment de la mise en oeuvre effective de la composition filmogène. Avec l'amidon et le plastifiant identifiés par la Demanderesse, il est possible de présenter la composition filmogène selon l'invention sous la forme d'un prêt-à-l'emploi, si bien qu'une étape de mélange extemporanée de l'amidon et du plastifiant, lors par exemple du pelliculage de comprimés, n'est plus nécessaire.

Une fois sous la forme d'une composition aqueuse, c'est-à-dire sous la forme d'une solution ou d'une suspension, la composition filmogène aqueuse selon l'invention présente en plus l'avantage d'une faible viscosité. Il est ainsi possible d'augmenter sa matière sèche et donc parallèlement de réduire son contenu en eau. Il en résulte que les films obtenus sont plus faciles à sécher, et les temps et coûts nécessaires à leur fabrication sont significativement réduits.La présente invention a ainsi pour premier objet une composition filmogène, en particulier destinée au pelliculage de formes solides, caractérisée en ce qu'elle comprend un amidon hydroxypropylé fluidifié et du sorbitol.

On entend par « composition filmogène » au sens de la présente invention, une composition comprenant au moins un polymère (agent filmogène), apte à former un film essentiellement continu en présence d'un solvant, en particulier d'eau. Il s'agit en particulier d'une composition filmogène amylacée, c'est-à-dire utilisant un amidon comme agent filmogène.

On entend par « amidon » au sens de la présente invention, l'amidon extrait, ou en d'autres termes, isolé, par toute technique adaptée et connue de l'homme du métier, d'une source amylacée, telle que les légumineuses, les céréales, les tubercules. L'amidon pourra par exemple être un amidon de pois, de maïs, de pomme-de-terre, ou de leurs mélanges.

On entend par « amidon hydroxypropylé » au sens de la présente invention, un amidon substitué par des groupements hydroxypropyle par toute technique connue de l'homme du métier, par exemple par réaction d'éthérification avec l'oxyde de propylène, présentant en particulier une teneur en groupements hydroxypropyle comprise entre 0,1 et 50% en poids sec par rapport au poids sec d'amidon hydroxypropylé, préférentiellement entre 1 et 15%, plus préférentiellement entre 5 et 9%, et en particulier proche de 7%. Cette teneur est en particulier déterminée par spectrométrie par Résonance Magnétique Nucléaire du proton, en particulier selon la norme EN ISO 11543:2002 F.

On entend par « amidon fluidifié » au sens de la présente invention, un amidon ayant subi une opération d'hydrolyse, c'est-à-dire une opération visant à réduire sa masse moléculaire moyenne. L'homme du métier sait comment obtenir de tels amidons, par exemple par des traitements chimiques tels que l'oxydation et les traitements acides, ou encore par des traitements enzymatiques. L'homme du métier ajustera naturellement le niveau de fluidification de l'amidon, en fonction de la viscosité souhaitée pour la composition filmogène.

Préférentiellement l'amidon hydroxypropylé fluidifié selon l'invention a une masse moléculaire moyenne en poids inférieure à 2 000 000 Da, préférentiellement supérieure à 20 000 Da, préférentiellement comprise entre 100 000 et 1 000 000 Da, tout préférentiellement entre 500 000 et 600 000 Da.

Cette masse moléculaire moyenne en poids peut être déterminée par l'homme du métier à l'aide d'une chromatographie d'exclusion stérique de type HPSEC-MALLS (High Performance Size Exclusion Chromatography coupled on-line with Multiple Angle Laser Light Scattering).

On peut mesurer cette masse par chromatographie d'exclusion stérique, selon le protocole suivant :
- préparation d'un échantillon par solubilisation de l'amidon hydroxypropylé fluidifié, en chauffant à 100°C pendant 30 min dans un solvant de dilution constitué d'un mélange DMSO / NaNO₃ (0,1M de NaNO₃ dans du DMSO), ledit échantillon pouvant présenter une concentration allant de 2 à 10 mg d'amidon par mL de solvant de dilution ;
- utilisation d'un appareil de chromatographie liquide à haute performance (CLHP) équipé d'une pompe, fonctionnant en mode isocratique, faisant circuler un solvant d'élution à 0,3 mL/min, d'un réfractomètre, d'un détecteur laser multiple angle light scattering de 18 angles chauffé à 35°C, par exemple un détecteur DAWN HELEOS de la société Wyatt, et d'un four de thermostatisation des colonnes chauffé à 35°C, par exemple équipé de colonnes de polyhydroxyméthacrylate de type SUPREMA et dont le solvant d'élution est par exemple une solution aqueuse de nitrate de sodium à 0,1 M, contenant 0,02 % en masse d'azide de sodium ;
- injection dans l'appareil d'un volume d'échantillon d'environ 100 µL.
Les masses moléculaires moyennes en poids peuvent être déterminées à partir des spectres obtenus, par exemple, en retraitant les spectres en exponentiel 1er ordre, à l'aide d'un logiciel d'analyse de type ASTRA v.5.Les poids moléculaires sont classiquement distribués selon une courbe gaussienne, le poids moléculaire moyen en poids étant proche de la médiane.

La composition filmogène selon l'invention est préférentiellement caractérisée en ce que l'amidon hydroxypropylé fluidifié est un amidon de légumineuse, et/ou un amidon de maïs.

Plus préférentiellement, l'amidon hydroxypropylé fluidifié selon l'invention est un amidon de légumineuse, préférentiellement de pois, plus préférentiellement de pois lisse.

Lorsque l'amidon selon l'invention est un amidon de pois, notamment de pois lisse, l'amidon a en particulier une teneur en amylose comprise entre 25 et 45%, de préférence entre 30 et 44%, encore plus préférentiellement entre 35 et 40%, et mieux encore entre 35 et 38%, ces pourcentages étant exprimés en poids sec par rapport au poids sec d'amidon de pois, et déterminés avant fluidification et hydroxypropylation dudit amidon.

Préférentiellement, la composition filmogène selon l'invention est caractérisée en ce que le sorbitol est compris dans ladite composition filmogène à une teneur comprise entre 5 et 80% en poids sec par rapport au poids sec d'amidon hydroxypropylé fluidifié, préférentiellement comprise entre 5 et 40%, et plus préférentiellement entre 15 et 35%, la borne à 35% étant préférentiellement exclue.

Il est à noter que dans la présente demande, les teneurs en les différents composés sont exprimées en fonction de la matière sèche totale de la composition filmogène, à l'exception des teneurs en sorbitol, qui, sauf indication contraire, sont exprimées en fonctions du poids sec d'amidon. En effet, c'est le rapport entre l'agent filmogène et l'agent plastifiant qui détermine notamment la qualité de la plastification et donc du film formé.

Avantageusement, la composition filmogène selon l'invention est caractérisée en ce que l'amidon hydroxypropylé fluidifié et le sorbitol représentent ensemble entre 15 et 100% en poids sec par rapport au poids total de la matière sèche de ladite composition filmogène, préférentiellement entre 30 et 95%, et plus préférentiellement entre 60 et 95%.

Préférentiellement, la composition filmogène selon l'invention est caractérisée en ce qu'il s'agit d'un prêt-à-l'emploi.

On entend par « prêt-à-l'emploi » au sens de la présente invention, une composition pulvérulente, en particulier destinée au pelliculage de formes solides. Le prêt-à-l'emploi peut être réalisé par tout moyen de mélange adapté et connu de l'homme du métier, par l'exemple à l'aide d'un mélangeur de poudre.

Dans un second mode de réalisation avantageux, la composition filmogène selon l'invention est caractérisée en ce qu'il s'agit d'une composition aqueuse.

La composition filmogène aqueuse selon l'invention est préférentiellement caractérisée en ce qu'elle présente une matière sèche comprise entre 5 et 50% en poids, préférentiellement entre 10 et 35%, plus préférentiellement entre 15 et 30%.

Elle présente avantageusement une viscosité mesurée à 25°C inférieure ou égale à 500 mPa.s, plus préférentiellement comprise entre 50 et 500 mPa.s, plus préférentiellement entre 80 et 300 mPa.s.

Cette viscosité, dans la présente invention, est une viscosité Brookfield déterminée au moyen d'un viscosimètre Brookfield RVF 100, en utilisant le mobile de l'appareil qui donne une lecture comprise entre 20 et 80% de l'échelle du cadran de l'appareil, à une vitesse de rotation de 100 tours par minute.

De manière générale, et de façon tout à fait avantageuse, la composition filmogène aqueuse ou le prêt-à-l'emploi selon l'invention, comprennent des additifs classiquement employés par l'homme du métier, en particulier dans le domaine du pelliculage, notamment choisis parmi :
- les antiagrégeants, en particulier des corps gras tels que les dérivés du glycérol, par exemple le monostéarate de glycérol, les sucro-esters, le stéarate de magnésium, le Kaolin, l'acide stéarique ;
- les opacifiants, en particulier les matières minérales telles que le dioxyde de titane ou le carbonate de calcium ;
- les pigments et colorants, par exemple des colorants solubles ou laques d'aluminium tels que E129, E132, E133, E110 ou E102, des pigments comme les oxydes de fer, des colorants d'origine naturelle tels que l'acide carminique ou un complexe cuivre-chlorophylle ;
- les arômes ou les édulcorants, par exemple de l'huile de menthe, l'aspartame, l'acésulfame ;
- les composés pour améliorer le brillant, par exemple des triglycérides à moyennes chaînes.

Préférentiellement, la composition filmogène selon l'invention comprend au moins un additif antiagrégant, préférentiellement de l'acide stéarique.

Préférentiellement, la composition filmogène selon l'invention comprend au moins un additif opacifiant, préférentiellement du dioxyde de titane.

La composition filmogène selon l'invention peut par ailleurs comprendre :
- d'autres agents filmogènes que l'amidon hydroxypropylé fluidifié utilisé conformément à l'invention, par exemple des polymères synthétiques tels que l'hydroxypropyl méthyl-cellulose (HPMC), l'hydroxypropyl-cellulose (HPC) ou l'alcool polyvinylique (PVA),
- et/ou d'autres plastifiants que le sorbitol selon l'invention, par exemple des dérivés de sorbitol, notamment des anhydrides de sorbitol, du glycérol, du polyéthylène glycol, du triéthylcitrate, du polysorbate, de la cire de Carnauba, de l'huile de ricin hydrogénée.

Préférentiellement, la composition filmogène selon l'invention comprend moins de 80% d'agent filmogène autre que l'amidon hydroxypropylé fluidifié selon l'invention, préférentiellement moins de 70%, préférentiellement moins de 60%, préférentiellement moins de 50%, préférentiellement moins de 40%, préférentiellement moins de 30%, préférentiellement moins de 20%, préférentiellement moins de 10% ; ces pourcentages étant exprimés en poids sec, par rapport au poids sec total d'agent filmogène de la composition filmogène.

De la même manière, le sorbitol selon l'invention étant à lui seul un excellent plastifiant de l'amidon hydroxypropylé fluidifié selon l'invention, la présence d'autres plastifiants est facultative. Ainsi préférentiellement, la composition filmogène selon l'invention comprend moins de 80% de plastifiants autres que le sorbitol, préférentiellement moins de 70%, préférentiellement moins de 60%, préférentiellement moins de 50%, préférentiellement moins de 40%, préférentiellement moins de 30%, préférentiellement moins de 20%, préférentiellement moins de 10% ; ces pourcentages étant exprimés en poids sec, par rapport au poids sec total de plastifiant de la composition filmogène.

Pour des raisons de facilité de mise en oeuvre, il est préférable de minimiser la quantité de matières premières à manipuler.

Ainsi, préférentiellement, l'amidon hydroxypropylé fluidifié et le sorbitol représentent dans la composition filmogène selon l'invention, l'essentiel, et préférentiellement la totalité, des agents filmogènes et des plastifiants respectivement.

L'amidon hydroxypropylé fluidifié de la composition filmogène selon l'invention peut en outre présenter d'autres modifications, et pourra par exemple avoir subi des traitements physiques, notamment choisis parmi les opérations connues de précuisson, de cuisson, d'extrusion, d'atomisation ou de séchage, les opérations de traitement par micro-ondes ou ultrasons, de plastification ou de granulation.

En particulier, l'amidon après hydroxypropylation et fluidification comprend souvent une part d'amidon restée sous forme granulaire, c'est-à-dire qui ne participe pas nécessairement à la formation du film. Il sera donc préférable de rendre soluble dans l'eau cette part d'amidon, afin que la composition filmogène contienne un maximum d'amidon disponible pour la formation du film.

Ainsi, de façon tout à fait avantageuse, l'amidon selon l'invention est caractérisé en ce qu'il est rendu soluble. Il peut être rendu soluble par toute technique connue de l'homme du métier, notamment par traitement thermique et/ou mécanique, par exemple par une opération de cuisson en milieu aqueux, éventuellement suivie d'une étape de séchage lorsque l'obtention d'un produit pulvérulent est souhaitée. L'opération visant à rendre soluble l'amidon peut tout à fait intervenir avant l'introduction dudit amidon dans la composition filmogène, de manière indifférente avant ou après l'hydroxypropylation et/ou la fluidification de l'amidon, ou encore après son introduction dans la composition filmogène, par exemple en cuisant la composition filmogène au moment de sa mise en oeuvre.

Bien que le sorbitol selon l'invention soit préférentiellement un sorbitol pulvérulent, par exemple tel que ceux commercialisés par la Demanderesse sous le nom NEOSORB® P60W, il pourra également s'agir d'un sorbitol liquide, cristallisable ou non.

La composition filmogène conforme à l'invention est utilisée avantageusement pour le pelliculage de formes solides.

On entend par « forme solide » au sens de la présente invention toute présentation de substances alimentaires, pharmaceutiques, cosmétiques, chimiques ou agrochimiques destinée à subir une opération de pelliculage. Des exemples de formes solides sont les comprimés, gélules, capsules, pellets, microsphères, granules, semences, les formes solides alimentaires telles que les biscuits, les céréales pour petit-déjeuner, les confiseries (chewing-gums, sucres-cuits, gélifiés), ou encore les produits sous forme de poudres et/ou de cristaux. Lorsque ces formes solides sont pelliculées, elles incluent alors dans leur structure au moins une couche de pelliculage, et la forme solide initiale est alors désignée par le terme « noyau ».

Pour la réalisation du pelliculage de formes solides, on peut utiliser toute technique connue de l'homme du métier, telle que la pulvérisation en lit fluidisé, ou en turbine conventionnelle ou perforée.

Ainsi la présente invention a également pour objet un procédé de pelliculage, caractérisé en ce qu'il comprend :
- une étape a') de pulvérisation d'une composition filmogène aqueuse selon l'invention sur un lit de formes solides en mouvement,
- une étape b) de séchage des formes solides ainsi pelliculées, préférentiellement concomitante à l'étape a').

La présente invention a en particulier pour objet un procédé de pelliculage, caractérisé en ce qu'il comprend :
- une étape a) de fabrication d'une composition filmogène aqueuse selon l'invention comprenant le mélange d'un prêt-à-l'emploi selon l'invention avec de l'eau,
- une étape a') de pulvérisation de la composition filmogène aqueuse obtenue à l'étape a) sur un lit de formes solides en mouvement.
- une étape b) de séchage des formes solides ainsi pelliculées, préférentiellement concomitante à l'étape a').

Comme montré dans les Exemples 2 à 3, la composition filmogène aqueuse peut tout à fait être pulvérisée sur un lit de comprimés préchauffé à une température de l'ordre de 35°C. Par opposition, dans les procédés décrits dans le brevet FR 2 862 654 B1, les compositions filmogènes plastifiées avec le glycérol étaient pulvérisées sur un lit de comprimé préchauffé à 55°C. Ceci est particulièrement avantageux puisque cela signifie que la température de travail peut être significativement abaissée. Il en résulte qu'en plus des réductions des coûts énergétiques, il est possible de pelliculer sans problème des comprimés sensibles à la température.

Ainsi, préférentiellement, le procédé selon l'invention est caractérisé en ce que le lit de formes solides est préchauffé à une température comprise entre 30 et 60°C, préférentiellement entre 30 et 40°C, en particulier de l'ordre de 35°C.

Préférentiellement, le gain de masse des formes solides obtenues après pelliculage est compris entre 1 et 10 % préférentiellement entre 2 et 6%.

La présente invention a également pour objet une forme solide comprenant un noyau recouvert d'au moins une couche de pelliculage, caractérisé en ce que cette couche de pelliculage comprend un amidon hydroxypropylé fluidifié et du sorbitol.

Avantageusement, l'amidon hydroxypropylé fluidifié est un amidon de légumineuse, et/ou un amidon de maïs, préférentiellement un amidon de pois, plus préférentiellement de pois lisse.

De façon particulièrement avantageuse, le sorbitol est compris dans la couche de pelliculage à une teneur comprise entre 5 et 80% en poids sec par rapport au poids sec d'amidon hydroxypropylé fluidifié, préférentiellement comprise entre 5 et 40%, plus préférentiellement entre 15 et 35%, la borne à 35% étant préférentiellement exclue.

Avantageusement, l'amidon hydroxypropylé fluidifié et le sorbitol représentent ensemble entre 15 et 100% en poids sec par rapport au poids total de la matière sèche de la couche de pelliculage, préférentiellement entre 30 et 95%, plus préférentiellement entre 60 et 95%.

Avantageusement, la couche de pelliculage de la forme solide selon l'invention possède une épaisseur moyenne comprise entre 10 et 90 µm, plus préférentiellement entre 20 et 60 µm, et plus préférentiellement encore entre 25 et 55µm.

Avantageusement, le noyau de la forme solide pelliculée est un comprimé.

Le noyau selon l'invention comprend préférentiellement au moins un ingrédient d'intérêt, notamment agricole, alimentaire, cosmétique ou pharmaceutique.

La composition filmogène selon l'invention convient aussi particulièrement pour la réalisation de films.

On entend ici par « film » un produit fin et plat, ayant une surface essentiellement plane, et une épaisseur comprise entre 5 et 3000 µm, préférentiellement entre 20 et 200 µm, plus préférentiellement entre 50 et 90 µm, et dont l'épaisseur est relativement faible en comparaison de sa longueur et de sa largeur. Ce sont en particulier des films préhensibles avec les doigts. Cela inclut des films alimentaires, pharmaceutiques ou cosmétiques. Il s'agit préférentiellement de films orodispersibles, mais il pourra également s'agir de films destinés à toute autre application dans laquelle l'obtention d'un film d'une telle épaisseur est avantageuse, par exemple de films avec support tels que les patchs transdermiques permettant l'administration d'un principe actif par application du patch sur la peau, de films pharmaceutiques ou cosmétiques topiques à poser sur la peau tels quels des films destinés à être dissous, par exemple dans des compositions cosmétiques, des aliments, des boissons.

Ainsi la présente invention a également pour objet un procédé de fabrication de films, caractérisé en ce qu'il comprend une étape a) d'étalement d'une composition filmogène aqueuse selon l'invention, et une étape b) de séchage de la solution ainsi étalée.

L'étape a) peut être réalisée par toute technique connue de l'homme du métier, par exemple par étalement d'une épaisseur faible et constante de la solution sur une surface plane ou cylindrique, suivi d'un séchage à température ambiante ou à chaud.

La présente invention a également pour objet un film, caractérisé en ce qu'il comprend un amidon hydroxypropylé fluidifié et du sorbitol.

Avantageusement, l'amidon hydroxypropylé fluidifié est un amidon de légumineuse, et/ou un amidon de maïs, préférentiellement un amidon de pois, plus préférentiellement de pois lisse.

De façon particulièrement avantageuse, le sorbitol est compris dans le film, à une teneur comprise entre 5 et 80% en poids sec par rapport au poids sec d'amidon hydroxypropylé fluidifié, préférentiellement comprise entre 5 et 40%, plus préférentiellement entre 15 et 35%, la borne à 35% étant préférentiellement exclue.

Avantageusement, l'amidon hydroxypropylé fluidifié et le sorbitol représentent ensemble entre 15 et 100% en poids sec par rapport au poids total de la matière sèche du film, préférentiellement entre 30 et 95%, plus préférentiellement entre 60 et 95%.

La composition selon l'invention peut par ailleurs tout à fait servir à la préparation de capsules molles, de gélules dures ou de « caplets ».

La présente invention a donc aussi pour objet un procédé de fabrication de têtes et/ou corps de gélules, et/ou un procédé de fabrication de capsules molles, caractérisé en ce qu'il comprend une étape de formage à l'aide d'une composition filmogène aqueuse selon l'invention.

La présente invention a également pour objet un procédé de fabrication de caplets, caractérisé en ce qu'il comprend l'immersion de formes solides dans une composition filmogène aqueuse selon l'invention.

Pour la fabrication de gélules, l'on peut utiliser un équipement conventionnel qui consiste à plonger des doigts métalliques dans la composition filmogène aqueuse maintenue à température constante. Pour la fabrication de capsules molles, l'on peut utiliser les techniques connues de formage sur tambours, ou par extrusion. Pour la fabrication de « caplets », l'on procède par exemple à l'immersion des comprimés mécaniquement ou à la main dans un bain contenant la composition filmogène aqueuse selon l'invention.

Les exemples ci-après font partie intégrante de la présente invention et servent à l'illustrer sans néanmoins en constituer une limitation.

### EXEMPLES

### EXEMPLE 1 - EFFET PLASTIFIANT DU SORBITOL SUR UN AMIDON HYDROXYPROPYLE FLUIDIFIE

Plusieurs compositions filmogènes à 10% de matières sèches sont réalisées. Pour chaque composition, 3 g sont prélevés et coulés en boîte de pétri de 55 mm de diamètre. Pour le séchage, les films sont laissés 72h à température ambiante.

Pour analyser la qualité de la plastification, la température de transition vitreuse (Tg) de chaque film est mesurée après stockage de ceux-ci à 66% d'humidité relative (HR) dans un dessiccateur comprenant une solution sursaturée en NaNO₂.

Pour la mesure de la Tg, les films sont pesés dans des creusets en aluminium de 40 µL non percés, et sont analysés par Analyse Thermique Différentielle (ATD), à l'aide de l'appareil TA Instruments Q20, selon les cycles de chauffage de -90°C à 120°C, à 10°C/min, et de refroidissement de 120°C à -90°C, à 20°C/min.

Les résultats de Tg obtenus sont présentés dans le tableau suivant :

| | Teneur en plastifiant (sorbitol ou glycérol) utilisée pour la réalisation du film, en poids sec par rapport au poids sec d'amidon | | | | | | | |
|---|---|---|---|---|---|---|---|---|
| | 0% | 2,5% | 5% | 7% | 10% | 20% | 30% | 40% |
| **Amidon de pois hydroxypropylé fluidifié + Sorbitol** | **67°C** | **65°C** | **39°C** | **3°C** | **-36°C** | **-34°C** | **-37°C** | **-42°C** |
| Amidon de pois hydroxypropylé fluidifié + Glycérol | 67°C | 66°C | 67°C | 51°C | 34°C | 19°C | 4°C | -22°C |
| Amidon de pois natif + Sorbitol | 75°C | 73°C | 75°C | 74°C | 59°C | 38°C | 21°C | 10°C |
| Amidon de pois hydroxypropylé + Sorbitol | 74°C | 75°C | 75°C | 68°C | 41°C | 19°C | -3°C | -21°C |

L'amidon de pois hydroxypropylé fluidifié est ici un amidon prégélatinisé présentant une teneur en groupements hydroxypropyle de 6,8%.

Le rôle du plastifiant est d'abaisser la Tg du film formé par l'agent filmogène. Pour un amidon hydroxypropylé fluidifié, l'addition de 5% de sorbitol en poids sec par rapport au poids sec d'amidon, suffit à diminuer la Tg.

Ainsi, contrairement à ce qu'enseigne l'art antérieur, le sorbitol permet la plastification de l'amidon même à de faibles teneurs, i.e. de l'ordre de 5% en poids sec par rapport au poids d'amidon sec.

Par ailleurs, ces performances ne sont pas retrouvées dès lors que l'on modifie la nature du plastifiant ou de l'amidon employé. Ainsi, aucune diminution de la Tg n'est observée dans les exemples comparatifs, pour des teneurs de l'ordre de 5% de plastifiant.

Enfin, même au-delà de 5% de plastifiant, en particulier, entre 5 et 40%, c'est toujours la composition filmogène selon l'invention qui permet d'obtenir les meilleures performances, i.e. la Tg la plus basse.

Ainsi, la composition filmogène selon l'invention sera avantageusement utilisée à des teneurs en sorbitol de l'ordre de 5% en poids sec par rapport au poids sec d'amidon hydroxypropylé fluidifié, en particulier entre 10 et 40%.

Cette étude montre clairement que les excellents résultats obtenus avec les compositions selon l'invention sont le fait du choix spécifique du couple agent filmogène / plastifiant identifié par la Demanderesse, en particulier parce que l'amidon choisi est un amidon fluidifié hydroxypropylé, et que le plastifiant choisi est le sorbitol.

### EXEMPLE 2 - PELLICULAGE DE COMPRIMES MULTIVITAMINES

### Exemple 2.a) - Comparaison de compositions filmogènes selon l'invention avec une composition filmogène utilisant le glycérol comme plastifiant

Trois compositions filmogènes A, B et C sont réalisées comme suit :

| | A | B | C |
|---|---|---|---|
| Amidon de pois hydroxypropylé fluidifié prégélatinisé (teneur en groupements hydroxypropyle = 6,8%) | 58% | 58% | 58% |
| Mélange pigment / opacifiant (Spectracol Yellow, Sensient) | / | 22% | 22% |
| Dioxyde de titane (Kronos, Kronos Titan GmbH) | 22% | / | / |
| Acide stéarique (Stéarinerie Dubois) | 15% | 15% | 15% |
| Sorbitol (Neosorb® P60W, Roquette Frères) | 5% (*soit 8,6% en poids sec par rapport au poids sec d'amidon*) | | / |
| Glycérol (G6279-1L, Sigma Aldrich) | / | | 5% |

les pourcentages étant exprimés en poids sec par rapport au poids total de la matière sèche.

De l'eau déminéralisée est ensuite ajoutée de manière à obtenir des compositions à 20% de matière sèche.

La viscosité Brookfield des compositions A et B mesurée est comprise entre 100 et 150 mPa.s.

Des comprimés multivitaminés minéraux ronds biconvexes, présentant un poids moyen de 600 mg, un diamètre moyen de 11,3 mm et une épaisseur moyenne de 6,3 mm, sont pelliculés avec la composition A, B, ou C.

Le pelliculage est réalisé par pulvérisation de la composition aqueuse à l'aide de l'équipement suivant :
- Turbine pleine: Dumoulin TW 360 (30cm de diamètre), équipée d'un système de soufflage d'air chaud et d'un système d'aspiration
- Buse: Binks 460
- Pompe péristaltique: modèle Watson Marlow 323, Tête de pompe: 313 DW (3 galets)
- Tube exacanal (diam int: 2mm, diam ext: 6 mm)
- 8 barres anti-glissement

Les conditions opératoires sont présentées dans le tableau suivant :

| | A | B | D |
|---|---|---|---|
| Quantité de comprimés (Kg) | 1 | | |
| Vitesse de rotation (rpm) | 16 | | |
| Nombre de pistolets de pulvérisation | 1 | | |
| Pression d'air de service (bar) | 4 | | |
| Température d'entrée d'air (°C) | 55 | 20 | 55 |
| Température du lit de comprimé (°C) | 36-38 | 20-22 | 36-38 |
| Débit de pulvérisation (g/min) | 2-3,4 | 1,25-2 | 2-3,4 |
| Temps de pulvérisation (min) | 72 | 121 | 70 |
| Gain de masse (%) | 5 | 5 | 3 |

Les comprimés pelliculés sont placés dans des conditions favorisant leur déformation. Ils sont incubés sans emballage à 40°C à 75% d'humidité relative (75%HR).

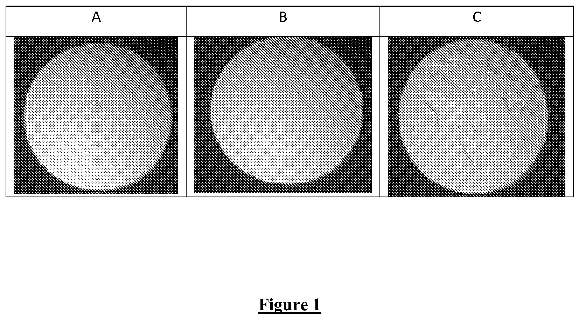

Les résultats sont présentés Figure 1, les comprimés A, B, et C, correspondant aux comprimés pelliculés avec les compositions A, B, et C respectivement.

Après un jour d'incubation, de larges fissurations des films de pelliculage ont été constatées pour les comprimés pelliculés avec la composition C. Aucune fissuration ne s'est produite sur les comprimés pelliculés avec les compositions A ou B selon l'invention, même après 5 jours d'incubation.

Les compositions filmogènes A et B présentent une teneur en sorbitol de 8,6% en poids par rapport au poids d'amidon hydroxypropylé fluidifié, le sorbitol étant le seul plastifiant de la composition filmogène.

Or, les comprimés pelliculés au moyen de ces compositions présentent un pelliculage tout à fait satisfaisant, uniforme et adhérent, contrairement à ce qui est constaté avec une composition utilisant les mêmes teneurs en glycérol.

Par ailleurs, la Demanderesse a également réalisé une composition dans laquelle l'amidon de pois hydroxypropylé fluidifié de la composition A était remplacé par une teneur égale en amidon hydroxypropylé (teneur en groupements hydroxypropyle = 6,8%), mais non fluidifié. Ces compositions se sont avérées impossibles à mettre en oeuvre, car elles présentaient une viscosité beaucoup trop élevée.

Ces essais confirment ainsi les résultats présentés dans l'Exemple 1, pour des applications dans le pelliculage, et notamment (i) la supériorité du sorbitol par rapport au glycérol (ii) son utilisation possible à des teneurs inférieures à 10%, en particulier de l'ordre de 8,6%, lorsque l'amidon à plastifier est un amidon hydroxypropylé fluidifié.

### Exemple 2.b) - Comparaison d'une composition filmogène selon l'invention utilisant un mélange sorbitol/glycérol comme plastifiant, avec des compositions filmogènes comprenant un mélange glycérol/lécithine

Plusieurs compositions filmogènes sont réalisées comme suit :

| | A | B | C |
|---|---|---|---|
| | | | |
| Amidon de pois hydroxypropylé fluidifié prégélatinisé (teneur en groupements hydroxypropyle = 6,8%) | 52,7 | 62 | 52,7 |
| Acide stéarique (Stéarinerie Dubois) | 10,2 | 12 | 10,2 |
| Dioxyde de titane (Kronos, Kronos Titan GmbH) | 12,75 | 15 | 12,75 |
| Oxyde de fer jaune (Neelikon Food Dyes and Chemicals Ltd) | 0,85 | 1 | 0,85 |
| Lécithine de soja (Phospholipon S20, Lipoid) | 4,25 | 5 | 19.25 |
| Glycérol (G6279-1L, Sigma Aldrich) | 4,25 | 5 | 4,25 |
| Sorbitol (Neosorb® P60W, Roquette Frères) | 15 | / | / |

les pourcentages étant exprimés en poids sec par rapport au poids total de la matière sèche.

De l'eau déminéralisée est ensuite ajoutée de manière à obtenir des compositions à 20% de matière sèche.

Les trois compositions A, B, et C comprennent ainsi 8,1% de glycérol en poids sec par rapport au poids sec d'amidon.

La composition A selon l'invention comprend en plus 28,5% de sorbitol et 8,1% de lécithine, le sorbitol représentant environ 64% en poids sec de la totalité des plastifiants

La composition B diffère de la composition A en ce que le sorbitol est absent. La composition C diffère de la composition A en ce que la part de sorbitol a été remplacée par la lécithine.

Des comprimés multivitaminés minéraux oblongs, présentant un poids moyen de 914 mg, une longueur moyenne de 16,3 mm, une largeur moyenne de 9,3 mm, et une épaisseur moyenne de 7,0 mm, sont pelliculés avec la composition A, B, ou C.

Le pelliculage est réalisé par pulvérisation de la composition aqueuse avec le même équipement que celui de l'Exemple 2.a)

Les conditions opératoires sont présentées dans le tableau suivant :

| | |
|---|---|
| Quantité de comprimés (Kg) | 1.0 |
| Vitesse de rotation (rpm) | 12 |
| Nombre de pistolets de pulvérisation | 1 |
| Pression d'air de service (bar) | 4 |
| Température d'entrée d'air (°C) | 55 |
| Température du lit de comprimé (°C) | 32 |
| Débit de pulvérisation (g/min) | 2.9 |
| Temps de pulvérisation (min) | 51 |
| Gain de masse (%) | 3.0 |

Les comprimés pelliculés sont placés dans des conditions favorisant leur déformation. Ils sont incubés sans emballage à 40°C à 75%HR.

Après un jour d'incubation, de larges fissurations des films de pelliculage ont été constatées pour les comprimés pelliculés avec la composition B. De petites fissurations ont été observées sur les comprimés pelliculés avec la composition C après une semaine d'incubation. Aucune fissuration ne s'est produite sur les comprimés pelliculés avec la composition A selon l'invention, même après 2 semaines d'incubation.

Ces résultats démontrent l'intérêt de l'utilisation du sorbitol en mélange avec le glycérol pour plastifier un amidon hydroxypropylé fluidifié, comparativement à l'utilisation de la lécithine.

### EXEMPLE 3 - PELLICULAGE DE COMPRIMES DE MACA (FORTEMENT HYGROSCOPIQUES)

Pour le pelliculage de comprimés fortement hygroscopiques, on utilise des comprimés de Maca oblongs, présentant un poids moyen de 758 mg, une longueur moyenne de 16,6 mm, une largeur moyenne de 9,2 mm et une épaisseur moyenne de 7,0 mm. Après de multiples études, la Demanderesse a en effet réussi à mettre en évidence que les phénomènes récurrents de fissuration des films observés sur ces comprimés étaient dus à leur tendance à se déformer et à augmenter en volume de façon particulièrement importante.

Dans les essais qui suivent, les mêmes déformations (notamment en termes d'épaisseur, de longueur et de largeur) ont été mesurées pour les comprimés pelliculés, quelle que soit la composition filmogène testée. Ainsi, c'est bien la résistance du film qui est testée.

### Exemple 3.a) - Comparaison de compositions filmogènes selon l'invention avec des compositions filmogènes utilisant d'autres plastifiants

Plusieurs compositions filmogènes sont réalisées comme suit :

| | | A | B | C |
|---|---|---|---|---|
| Amidon de pois hydroxypropylé fluidifié prégélatinisée (teneur en groupements hydroxypropyle = 6,8%) | | 60% | 68,75% | 57.3% |
| Acide stéarique (Stéarinerie Dubois) | | 10% | / | / |
| Pigment nacré (Sensipearl gold, Sensient) | | 9,8% | 24% | 20% |
| Pigment jaune (Yellow lake, Silesia) | | 0,2% | 1% | 0,8% |
| Stéarate de magnesium végétal (Barlocher) | | / | 6,25% | 5,2% |
| Lécithine de soja (Phospholipon S20, Lipoid) | | / | / | 16,7% |
| Sorbitol (Neosorb® P60W, Roquette Frères) | En poids par rapport à la matière sèche | 20% | / | / |
| | *Soit en poids par rapport à l'amidon* | 33% | / | / |

les pourcentages étant exprimés, sauf indication contraire, en poids sec par rapport au poids total de la matière sèche.

De l'eau déminéralisée est ensuite ajoutée de manière à obtenir des compositions à 20% de matières sèches.

Le pelliculage des comprimés Maca avec les compositions A, B ou C est réalisé par pulvérisation de la composition aqueuse avec le même équipement que celui de l'Exemple 2.a).

Les conditions opératoires sont présentées dans le tableau suivant :

| | |
|---|---|
| Quantité de comprimés (Kg) | 1.0 |
| Vitesse de rotation (rpm) | 12 |
| Nombre de pistolets de pulvérisation | 1 |
| Pression de service (bar) | 4 |
| Température d'entrée d'air (°C) | 56 |
| Température du lit de comprimé (°C) | 35 |
| Débit de pulvérisation (g/min) | 2.9 |
| Temps de pulvérisation (min) | 51 |
| Gain de masse (%) | 3.0 |

Les comprimés pelliculés sont ensuite placés dans des conditions favorisant leur déformation. Ils sont incubés sans emballage à 40°C à 75%HR pendant 5 jours.

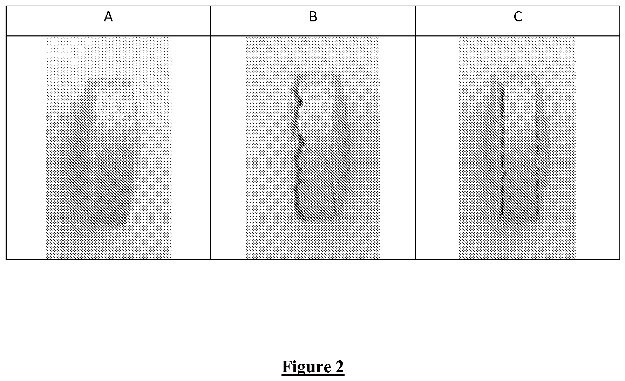

Les résultats obtenus sont présentés Figure 2, les comprimés A, B, et C correspondant aux comprimés pelliculés avec les compositions A, B, et C respectivement.

Au bout de quelques heures à peine, des fissurations des films de pelliculage ont été constatées pour les comprimés pelliculés avec les compositions B ou C, c'est-à-dire utilisant comme plastifiant le stéarate de magnésium, ou un mélange stéarate de magnésium / lécithine de soja, et comme agent filmogène un amidon hydroxypropylé fluidifié.

Ce phénomène n'est pas observé pour les comprimés pelliculés avec la composition A selon l'invention, qui comprend du sorbitol comme unique plastifiant.

La Demanderesse a par ailleurs réalisé d'autres essais, utilisant d'autres plastifiants pour des compositions filmogènes d'amidon de pois hydroxypropylé fluidifié.

Ces essais consistaient à remplacer le sorbitol de la composition A, par une quantité équivalente en poids d'autres composés. Les résultats obtenus peuvent être synthétisés de la façon suivante :
- le stéarate de magnésium, l'acide stéarique, le monostéarate de glycérol, le mygliol et la lécithine, n'ont pas permis de répondre aux problèmes techniques mentionnés dans la présente invention. En particulier, des fissurations des films sont apparues systématiquement sur les comprimés de Maca, avant la fin du premier jour d'incubation.
- les PEG 200, 400, 4000 et 6000, le stéarate de polyéthylène glycol, le triéthylcitrate, le polysorbate, la triacétine, le dibutyl sébaçate conduisent tous à la fissuration des films en cours de procédé de pelliculage des comprimés Maca, si bien que les compositions filmogènes étaient tout simplement impossibles à mettre en oeuvre.

### Exemple 3.b) - Comparaison d'une composition filmogène selon l'invention, avec une composition filmogène utilisant le mannitol comme plastifiant

Un prêt-à-l'emploi selon l'invention est réalisé comme suit (Prêt-à-l'emploi 1):
- Amidon de pois hydroxypropylé fluidifié prégélatinisé (teneur en groupements hydroxypropyle = 6,8%) : 77 %
- Acide stéarique (Stéarinerie Dubois) : 8 %
- Sorbitol (Neosorb® P60W, Roquette Frères) : 15%
les pourcentages étant exprimés en poids par rapport au poids de matières sèches.

Le prêt-à-l'emploi selon l'invention est comparé à un prêt-à-l'emploi (Prêt-à-l'emploi 2) de formulation :
- Amidon de maïs hydroxypropylé fluidifé prégélatinisé : 60 %
- Talc : 20 %
- Lécithine : 5 %
- Mannitol : 15 %
les pourcentages étant exprimés en poids par rapport au poids de matières sèches.

De l'eau déminéralisée et du dioxyde de titane sont ensuite ajoutés au Prêt-à-l'emploi 1 et 2, de manière à obtenir des compositions à 20% de matière sèche, le dioxyde de titane représentant 20% de la matière sèche totale.

Dès la réalisation de la composition filmogène aqueuse, on remarque que le Prêt-à-l'emploi 1 se dissout plus rapidement que le Prêt-à-l'emploi 2, et permet donc une mise en oeuvre plus aisée de la composition filmogène aqueuse.

Le pelliculage des comprimés de Maca est réalisé par pulvérisation de la composition aqueuse à l'aide de l'équipement suivant :
- Turbine perforée O'HARA Labcoat M
- Diamètre de la turbine de pelliculage : 8.5"
- 6 barres anti-glissement
- Pistolet de pulvérisation : Schlick 970/7-1 S75 (diamètre de la buse 0,8mm)
- Pompe péristaltique : Watson Marlow model 323, Head: 313 DW (3 rollers)
- Tube exacanal (diam int: 2mm, diam ext: 6mm)

Les conditions opératoires sont présentées dans le tableau suivant :

| | |
|---|---|
| Quantité de comprimés (Kg) | 0.3 |
| Vitesse de rotation (rpm) | 18 |
| Nombre de pistolets de pulvérisation | 1 |
| Pression d'atomisation (bar) | 1 |
| Pression d'écrasement du jet (bar) | 1 |
| Température d'entrée d'air (°C) | 50 |
| Température du lit de comprimé (°C) | 34 |
| Débit de pulvérisation (g/min) | 2 |
| Temps de pulvérisation (min) | 25 |
| Gain de masse (%) | 3,3 |

Les comprimés pelliculés sont placés dans des conditions favorisant leur déformation. Ils sont incubés sans emballage à 40°C à 75%HR.

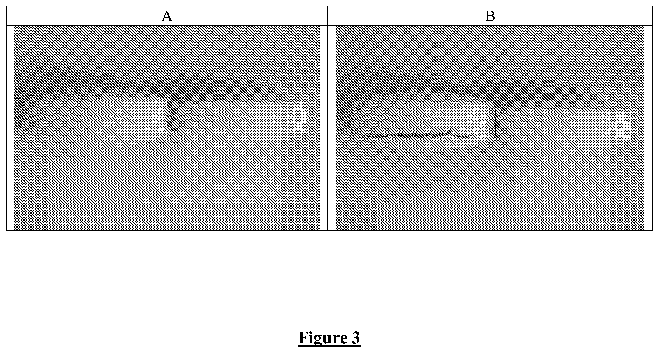

Les résultats obtenus sont présentés Figure 3, les comprimés A et B correspondant aux comprimés pelliculés avec les compositions A et B respectivement.

Des fissurations des films de pelliculage ont été constatées après 5 jours pour les comprimés pelliculés avec la composition B, qui ne se sont pas produites avec les comprimés pelliculés avec la composition A selon l'invention.

Ces résultats confirment la supériorité du sorbitol pour plastifier un amidon hydroxypropylé fluidifié, notamment par rapport à un autre polyol, le mannitol.

## Revendications

1. Composition filmogène, en particulier destinée au pelliculage de formes solides, **caractérisée en ce qu'**elle comprend un amidon hydroxypropylé fluidifié et du sorbitol et **en ce que** le sorbitol est compris dans ladite composition filmogène à une teneur comprise entre 5 et 80% en poids sec par rapport au poids sec d'amidon hydroxypropylé fluidifié, préférentiellement comprise entre 5 et 40%, plus préférentiellement entre 15 et 35%, la borne à 35% étant préférentiellement exclue.

2. Composition filmogène selon la revendication 1, **caractérisée en ce que** l'amidon hydroxypropylé fluidifié est un amidon de maïs et/ou de légumineuse, préférentiellement un amidon de pois, plus préférentiellement de pois lisse.

3. Composition filmogène selon l'une ou l'autre de la revendication 1 ou 2, **caractérisée en ce que** l'amidon hydroxypropylé fluidifié et le sorbitol représentent ensemble entre 15 et 100% en poids sec par rapport au poids total de la matière sèche de ladite composition filmogène, préférentiellement entre 30 et 95%, plus préférentiellement entre 60 et 95%.

4. Composition filmogène selon l'une quelconque des revendications 1 à 3, **caractérisée en ce qu'**elle comprend en outre un agent anti-agrégeant, préférentiellement de l'acide stéarique.

5. Composition filmogène selon l'une quelconque des revendications 1 à 4, **caractérisée en ce qu'**elle comprend en outre un agent opacifiant, préférentiellement du dioxyde de titane.

6. Composition filmogène selon l'une quelconque des revendications 1 à 5, **caractérisé en ce que** l'amidon hydroxypropylé fluidifié est rendu soluble.

7. Composition filmogène selon l'une quelconque des revendications 1 à 6, **caractérisée en ce qu'**il s'agit d'un prêt-à-l'emploi.

8. Composition filmogène selon l'une quelconque des revendications 1 à 6, **caractérisée en ce qu'**il s'agit d'une composition aqueuse.

9. Composition filmogène aqueuse selon la revendication 8, **caractérisée en ce qu'**elle présente une matière sèche comprise entre 5 et 50% en poids, préférentiellement entre 10 et 35%, plus préférentiellement entre 15 et 30%.

10. Composition filmogène aqueuse selon l'une ou l'autre des revendications 8 ou 9, **caractérisée en ce qu'**elle présente une viscosité Brookfield à 25°C inférieure ou égale à 500 mPa.s, plus préférentiellement comprise entre 50 et 500 mPa.s, plus préférentiellement entre 80 et 300 mPa.s.

11. Procédé de pelliculage, **caractérisé en ce qu'**il comprend :
- une étape a') de pulvérisation d'une composition filmogène aqueuse telle que définie dans l'une ou l'autre des revendications 8 à 10 sur un lit de formes solides en mouvement,
- une étape b) de séchage des formes solides ainsi pelliculées, préférentiellement concomitante à l'étape a').

12. Procédé de pelliculage, **caractérisé en ce qu'**il comprend :
- une étape a) de fabrication d'une composition filmogène aqueuse comprenant le mélange d'un prêt-à-l'emploi tel que défini dans la revendication 7 avec de l'eau,
- une étape a') de pulvérisation de la composition filmogène aqueuse obtenue à l'étape a) sur un lit de formes solides en mouvement,
- une étape b) de séchage des formes solides ainsi pelliculées, préférentiellement concomitante à l'étape a').

13. Procédé de pelliculage selon l'une ou l'autre des revendications 11 ou 12, **caractérisé en ce que** le lit de formes solides est préchauffé à une température comprise entre 30 et 60°C, préférentiellement entre 30 et 40°C, en particulier de l'ordre de 35°C.

14. Forme solide comprenant un noyau recouvert d'au moins une couche de pelliculage, **caractérisé en ce que** cette couche de pelliculage comprend un amidon hydroxypropylé fluidifié et du sorbitol, et **en ce que** le sorbitol est compris dans ladite couche de pelliculage à une teneur comprise entre 5 et 80% en poids sec par rapport au poids sec d'amidon hydroxypropylé fluidifié, préférentiellement comprise entre 5 et 40%, plus préférentiellement entre 15 et 35%, la borne à 35% étant préférentiellement exclue.

15. Film, en particulier orodispersible, **caractérisé en ce qu'**il comprend un amidon hydroxypropylé fluidifié et du sorbitol, et **en ce que** le sorbitol est compris dans ledit film à une teneur comprise entre 5 et 80% en poids sec par rapport au poids sec d'amidon hydroxypropylé fluidifié, préférentiellement comprise entre 5 et 40%, plus préférentiellement entre 15 et 35%, la borne à 35% étant préférentiellement exclue.

## Patentansprüche

1. Filmbildende Zusammensetzung, insbesondere geeignet zur Beschichtung fester Formen, **dadurch gekennzeichnet, dass** sie eine verflüssigte Hydroxypropylstärke und Sorbitol umfasst und dadurch, dass des Sorbitol in der filmbildenden Zusammensetzung in einer Menge zwischen 5 und 80% Trockengewicht, bezogen auf das Trockengewicht der verflüssigten Hydroxypropylstärke, enthalten ist, bevorzugt zwischen 5 und 40%, stärker bevorzugt zwischen 15 und 35%, wobei die Grenze 35% bevorzugt ausgeschlossen ist.

2. Filmbildende Zusammensetzung nach Anspruch 1, **dadurch gekennzeichnet, dass** die verflüssigte Hydroxypropylstärke eine Maisstärke und/oder Hülsenfruchtstärke ist, bevorzugt eine Erbsenstärke, stärker bevorzugt von Glatter Erbse.

3. Filmbildende Zusammensetzung nach Anspruch 1 oder 2, **dadurch gekennzeichnet, dass** die verflüssigte Hydroxypropylstärke und das Sorbitol zusammen zwischen 15 und 100% Trockengewicht, bezogen auf das Gesamtgewicht der Trockenmasse der filmbildenden Zusammensetzung, ausmachen, bevorzugt zwischen 30 und 95%, stärker bevorzugt zwischen 60 und 95%.

4. Filmbildende Zusammensetzung nach einem der Ansprüche 1 bis 3, **dadurch gekennzeichnet, dass** sie weiterhin ein Trennmittel umfasst, bevorzugt Stearinsäure.

5. Filmbildende Zusammensetzung nach einem der Ansprüche 1 bis 4, **dadurch gekennzeichnet, dass** sie weiterhin ein Trübungsmittel umfasst, bevorzugt Titandioxid.

6. Filmbildende Zusammensetzung nach einem der Ansprüche 1 bis 5, **dadurch gekennzeichnet, dass** die verflüssigte Hydroxypropylstärke löslich gemacht wird.

7. Filmbildende Zusammensetzung nach einem der Ansprüche 1 bis 6, **dadurch gekennzeichnet, dass** es sich um ein gebrauchsfertiges Produkt handelt.

8. Filmbildende Zusammensetzung nach einem der Ansprüche 1 bis 6, **dadurch gekennzeichnet, dass** es sich um eine wässrige Zusammensetzung handelt.

9. Wässrige filmbildende Zusammensetzung nach Anspruch 8, **dadurch gekennzeichnet, dass** sie zwischen 5 und 50 Gewichts-% Trockenmasse aufweist, bevorzugt zwischen 10 und 35 Gewichts-%, stärker bevorzugt zwischen 15 und 30 Gewichts-%.

10. Wässrige filmbildende Zusammensetzung nach Anspruch 8 oder 9, **dadurch gekennzeichnet, dass** sie bei 25°C eine Viskosität nach Brookfield von kleiner als oder gleich 500 mPa.s aufweist, stärker bevorzugt zwischen 50 und 500 mPa.s, stärker bevorzugt zwischen 80 und 300 mPa.s.

11. Beschichtungsverfahren, **dadurch gekennzeichnet, dass** es umfasst:
- einen Schritt a') der Zerstäubung einer wässrigen filmbildenden Zusammensetzung, wie in einem der Ansprüche 8 bis 10 definiert, auf ein bewegtes Bett fester Formen,
- einen Schritt b) des Trocknens der so beschichteten festen Formen, bevorzugt gleichzeitig mit Schritt a').

12. Beschichtungsverfahren, **dadurch gekennzeichnet, dass** es umfasst:
- einen Schritt a) der Herstellung einer wässrigen filmbildenden Zusammensetzung umfassend die Mischung aus einem gebrauchsfertigen Produkt, wie in Anspruch 7 definiert, mit Wasser,
- einen Schritt a') der Zerstäubung der in Schritt a) erhaltenen wässrigen filmbildenden Zusammensetzung, wie in einem der Ansprüche 8 bis 10 definiert, auf ein bewegtes Bett fester Formen,
- einen Schritt b) des Trocknens der so beschichteten festen Formen, bevorzugt gleichzeitig mit Schritt a').

13. Beschichtungsverfahren nach Anspruch 11 oder 12, **dadurch gekennzeichnet, dass** das Bett fester Formen auf eine Temperatur zwischen 30 und 60°C vorgewärmt wird, bevorzugt zwischen 30 und 40°C, insbesondere in Höhe von von 35°C.

14. Feste Form umfassend einen Kern, der mit mindestens einer Beschichtungsschicht überzogen ist, **dadurch gekennzeichnet, dass** diese Beschichtungsschicht eine verflüssigte Hydroxypropylstärke und Sorbitol umfasst und dadurch, dass das Sorbitol in der filmbildenden Zusammensetzung in einer Menge zwischen 5 und 80% Trockengewicht, bezogen auf das Trockengewicht der verflüssigten Hydroxypropylstärke, enthalten ist, bevorzugt zwischen 5 und 40%, stärker bevorzugt zwischen 15 und 35%, wobei die Grenze 35% bevorzugt ausgeschlossen ist.

15. Film, welcher insbesondere orodispersibel ist, **dadurch gekennzeichnet, dass** er eine verflüssigte Hydroxypropylstärke und Sorbitol umfasst und dadurch, dass das Sorbitol in der filmbildenden Zusammensetzung in einer Menge zwischen 5 und 80% Trockengewicht, bezogen auf das Trockengewicht der verflüssigten Hydroxypropylstärke, enthalten ist, bevorzugt zwischen 5 und 40%, stärker bevorzugt zwischen 15 und 35%, wobei die Grenze 35% bevorzugt ausgeschlossen ist.

## Claims

1. A film-forming composition, in particular intended for the film-coating of solid forms, **characterized in that** it comprises a fluidized hydroxypropyl starch and sorbitol and **in that** the sorbitol is included in said film-forming composition at a content of between 5% and 80% by dry weight relative to the dry weight of fluidized hydroxypropyl starch, preferentially between 5% and 40%, more preferentially between 15% and 35%, the limit at 35% being preferentially excluded.

2. The film-forming composition as claimed in claim 1, **characterized in that** the fluidized hydroxypropyl starch is a corn and/or legume starch, preferentially a pea starch, more preferentially a smooth pea starch.

3. The film-forming composition as claimed in either of claims 1 and 2, **characterized in that** the fluidized hydroxypropyl starch and the sorbitol together represent between 15% and 100% by dry weight relative to the total weight of the solids of said film-forming composition, preferentially between 30% and 95%, more preferentially between 60% and 95%.

4. The film-forming composition as claimed in any one of claims 1 to 3, **characterized in that** it also comprises an antiaggregating agent, preferentially stearic acid.

5. The film-forming composition as claimed in any one of claims 1 to 4, **characterized in that** it also comprises an opacifying agent, preferentially titanium dioxide.

6. The film-forming composition as claimed in any one of claims 1 to 5, **characterized in that** the fluidized hydroxypropyl starch is made soluble.

7. The film-forming composition as claimed in any one of claims 1 to 6, **characterized in that** it is a ready-to-use product.

8. The film-forming composition as claimed in any one of claims 1 to 6, **characterized in that** it is an aqueous composition.

9. The aqueous film-forming composition as claimed in claim 8, **characterized in that** it has a solids content of between 5% and 50% by weight, preferentially between 10% and 35%, more preferentially between 15% and 30%.

10. The aqueous film-forming composition as claimed in either of claims 8 and 9, **characterized in that** it has a Brookfield viscosity, at 25°C, of less than or equal to 500 mPa.s, more preferentially of between 50 and 500 mPa.s, more preferentially between 80 and 300 mPa.s.

11. A film-coating process, **characterized in that** it comprises:
- a step a') of spraying an aqueous film-forming composition as defined in one or another of claims 8 to 10 on to a moving bed of solid forms,
- a step b) of drying the solid forms thus film-coated, preferentially concomitant with step a').

12. A film-coating process, **characterized in that** it comprises:
- a step a) of producing an aqueous film-forming composition comprising mixing a ready-to-use product as defined in claim 7 with water,
- a step a') of spraying the aqueous film-forming composition obtained in step a) on to a moving bed of solid forms,
- a step b) of drying the solid forms thus film-coated, preferentially concomitant with step a').

13. The film-coating process as claimed in either of claims 11 and 12, **characterized in that** the bed of solid forms is preheated to a temperature of between 30 and 60°C, preferentially between 30 and 40°C, in particular of about 35°C.

14. A solid form comprising a core covered with at least one film-coating layer, **characterized in that** this film-coating layer comprises a fluidized hydroxypropyl starch and sorbitol, and **in that** the sorbitol is included in said film-coating layer at a content of between 5% and 80% by dry weight relative to the dry weight of fluidized hydroxypropyl starch, preferentially of between 5% and 40%, more preferentially between 15% and 35%, the limit at 35% being preferentially excluded.

15. A film, in particular an orodispersible film, **characterized in that** it comprises a fluidized hydroxypropyl starch and sorbitol, and **in that** the sorbitol is included in said film at a content of between 5% and 80% by dry weight relative to the dry weight of fluidized hydroxypropyl starch, preferentially of between 5% and 40%, more preferentially between 15% and 35%, the limit at 35% being preferentially excluded.
